(19) Europäisches Patentamt
European Patent Office
Office européen des brevets

(11) Veröffentlichungsnummer : **0 325 132 B1**

(12) # EUROPÄISCHE PATENTSCHRIFT

(45) Veröffentlichungstag der Patentschrift :
**21.08.91 Patentblatt 91/34**

(51) Int. Cl.[5] : **B01J 23/46,** B01J 23/64, B01J 23/58, C07C 209/68

(21) Anmeldenummer : **89100255.2**

(22) Anmeldetag : **09.01.89**

(54) **Rhodium-Katalysatoren, Verfahren zu ihrer Herstellung und Verfahren zur Herstellung von gegebenenfalls substituiertem Diphenylamin unter Einsatz der Rhodium-Katalysatoren.**

(30) Priorität : **22.01.88 DE 3801754**

(43) Veröffentlichungstag der Anmeldung :
**26.07.89 Patentblatt 89/30**

(45) Bekanntmachung des Hinweises auf die Patenterteilung :
**21.08.91 Patentblatt 91/34**

(84) Benannte Vertragsstaaten :
**BE CH DE FR GB IT LI**

(56) Entgegenhaltungen :
**EP-A- 0 053 817**
**EP-A- 0 123 233**
**EP-A- 0 208 933**
**FR-A- 2 367 035**

(73) Patentinhaber : **BAYER AG**
**W-5090 Leverkusen 1 Bayerwerk (DE)**

(72) Erfinder : **Immel, Otto, Dr.**
**Immenhofweg 26**
**W-4150 Krefeld (DE)**
Erfinder : **Schwarz, Hans-Helmut, Dr.**
**Rather-Strasse 90**
**W-4150 Krefeld 1 (DE)**

EP 0 325 132 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Rhodium-Katalysatoren, sowie ein Verfahren zu ihrer Herstellung. Diese neuen Rhodium-Katalysatoren eignen sich insbesondere zur Herstellung von gegebenenfalls substituiertem Diphenylamin aus entsprechend substituiertem Dicyclohexylamin, so daß die Erfindung weiterhin ihre Verwendung in einem Verfahren zur Herstellung von solchem gegebenenfalls substituiertem Diphenylamin unter Einsatz von Rhodium-Katalysatoren betrifft.

Zur Herstellung von Diphenylamin und dessen Derivaten ist aus DE-OS 2331878 ein Verfahren bekannt, bei dem man von Iminen, wie N-Cyclohexyliden-anilin und dessen Derivaten ausgeht und sie in der Gasphase in Gegenwart von Trägerkatalysatoren auf der Basis von Nickel, Platin, Palladium oder Kupfer-Chrom dehydriert. N-Cyclohexyliden-anilin wird beispielsweise aus Cyclohexanon und Anilin durch Kondensation hergestellt.

Weiterhin ist es aus DE-OS 2520893 bekannt, Diphenylamin durch katalytische Dehydrierung von Verbindungen und/oder Verbindungsgemischen, die aus ganz oder teilweise hydriertem Diphenylamin bestehen, in Gegenwart eines Nickel-Chrom, Aluminium, Kupfer, Mangan und Alkali enthaltenden Dehydrierungskatalysators herzustellen. Als solche Verbindungen werden zweikernige aromatische Imine in den Ausführungsbeispielen gezeigt.

Ein weiteres, aus Kinet, Katal. 28 (1), 250-254 (zitiert nach C.A. 107 (23), 217420 z) bekanntes Verfahren zeigt schließlich, daß an Hydrierungs-/Dehydrierungskatalysatoren außer der dehydrierenden Aromatisierung auch eine Cyclisierung zum Carbazol zu berücksichtigen ist. N-Cyclohexylanilin ergibt beispielsweise an Pt (1%)/$Al_2O_3$ bei 380°C 41% Carbazol und nur 18% Diphenylamin ; Dicyclohexylamin ergibt unter ähnlichen Bedingungen 41% Carbazol und nur 17% Diphenylamin und N-Cyclohexylidenanilin ergibt 40% Carbazol und 20% Diphenylamin. Daneben werden Entaminierungs- und Umlagerungsprodukte wie Anilin, Benzol, Diphenyl und 4-Amino-diphenyl beobachtet.

Die genannten Verfahren ergeben unzureichende Umsätze und Ausbeuten und sind in jeder Weise für ein industriell durchzuführendes Verfahren verbesserungsbedürftig. Der Wunsch, ein technisch einfach durchzuführendes Verfahren, welches hohe Ausbeuten sicherstellt und eine leichte Aufarbeitung des Reaktionsproduktes ermöglicht, bereitzustellen, wird durch die Erfindung befriedigt.

Die Erfindung betrifft Rhodium und mindestens ein anderes Platinmetall aus der Gruppe Palladium, Platin und Iridium enthaltende Katalysatoren auf Trägern, die die Edelmetalle in einer Gesamtmenge von 0,05-5 Gew.-%, bevorzugt 0,05-4 Gew.-%, besonders bevorzugt 0,1-3 Gew.-%, und einem Gewichtsanteil des Rhodiums an allen Edelmetallen von 10-90%, bevorzugt 15-80%, besonders bevorzugt 20-70%, enthalten und die weiterhin Zusätze von 1-6 Gew.-% eines Alkalihydroxids und 1-6 Gew.-% eines Alkalimetallsulfats enthalten, wobei alle Prozentsätze auf das Gesamtgewicht des Katalysators bezogen sind.

Ein wichtiges Merkmal des erfindungsgemäßen Katalysators ist die Kombination von Rhodium mit mindestens einem der genannten anderen Platinmetalle. In bevorzugter Weise wird Rhodium mit Palladium oder Platin oder einem Gemisch aus Palladium und Platin kombiniert. In besonders bevorzugter Weise wird zur Kombination mit dem Rhodium Palladium oder Platin allein eingesetzt.

Der erfindungsgemäße Katalysator enthält weiterhin 1-6 Gew.-%, bevorzugt 2-5 Gew.-%, bezogen auf das Gesamtgewicht des Katalysators eines Alkalihydroxids, wie Lithiumhydroxid, Natriumhydroxid, Kaliumhydroxid, Rubidiumhydroxid, Caesiumhydroxid, bevorzugt Lithiumhydroxid, Natriumhydroxid, Kaliumhydroxid, besonders bevorzugt Natriumhydroxid oder Kaliumhydroxid. Der erfindungsgemäße Katalysator enthält weiterhin in Kombination mit einem oder mehreren der genannten Alkalihydroxide zusätzlich 1-6 Gew.-%, bevorzugt 2-5 Gew.-%, bezogen auf das Gesamtgewicht des Katalysators eines Alkalimetallsulfats wie Lithiumsulfat, Natriumsulfat, Kaliumsulfat, Rubidiumsulfat, Caesiumsulfat, bevorzugt Lithiumsulfat, Natriumsulfat, Kaliumsulfat, besonders bevorzugt Natriumsulfat oder Kaliumsulfat.

Die genannten Bestandteile der erfindungsgemäßen Katalysatoren sind auf einem Träger angeordnet. Beispiele für solche Träger sind Aluminiumoxid, Aluminiumspinell, Aktivkohle, Kieselgur, Bentonit, Bimsstein, Silcagel, $ZrO_2$, $TiO_2$, ZnO, MgO sowie Oxide der Seltenen Erden.

Die genannten Bestandteile der erfindungsgemäßen Katalysatoren sind bevorzugt auf einem Träger aus Aluminiumoxid oder einem Aluminium-Spinell, besonders bevorzugt auf einem $Al_2O_3$ oder Al-Spinell, das mit Chrom und Mangan behandelt worden ist, aufgebracht. Als Aluminiumoxid kommen insbesondere die $\alpha$- und die $\gamma$-Modifikation in Frage. Aluminium-Spinelle sind Verbindungen der Formel $Me(II)Al_2O_4$ bzw. $Me(I)AlO_2$, in denen Me(II) ein zweiwertiges Metallkation des Eisens, Zinks, Nickels, Kupfers, Kobalts, Cadmiums, Magnesiums oder anderer, bevorzugt des Magnesiums, und Me(I) ein einwertiges Kation, beispielsweise Lithium (Lithium-Aluminium-Spinell), ist. Das Aluminium in den Spinellen kann teilweise durch dreiwertiges Eisen, Chrom oder Mangan ersetzt sein. In bevorzugter Weise wird $Al_2O_3$, besonders bevorzugt das $\gamma$-$Al_2O_3$, eingesetzt. Ein solcher Träger weist in der besonders bevorzugten Weise einen Gehalt an Chrom und Mangan von zusammen

etwa 0,05-8 Gew.-%, bevorzugt 0,2-5 Gew.-%, bezogen auf das Gesamtgewicht des Katalysators, auf. Das Gewichtsverhältnis von Chrom und Mangan beträgt etwa 5 : 1-1 : 5, bevorzugt 10 : 9-1 : 2. Solche mit Chrom und Mangan behandelten Träger sind aus EP 208933 bekannt.

Zur Herstellung der beschriebenen erfindungsgemäßen Katalysatoren kann in der besonders bevorzugten Weise so vorgegangen werden, daß auf ein $Al_2O_3$ oder einen Aluminium-Spinell in Form von Strangpreßlingen, Pillen oder Kugeln mit Abmessungen von etwa 2-10 mm Verbindungen des Chroms und Mangans aufgebracht werden, der so beaufschlagte Träger auf höhere Temperatur erhitzt wird, anschließend getrennt die Edelmetalle und eines oder mehrere Alkalihydroxide und eines oder mehrere Alkalisulfate aufgetragen werden ; nach jedem Auftragen wird eine Trocknung vorgenommen, im allgemeinen bei 100-140°C bei vermindertem bis normalem Druck, wie 1-1000 mbar, bevorzugt 10-500 mbar, beispielsweise bei Wasserstrahlvakuum.

Das Aufbringen des Chroms und Mangans auf den Katalysatorträger in der besonders bevorzugten Art kann beispielsweise durch gemeinsames Ausfällen eines Mangan-Chrom-Hydroxidgemisches aus einer Chrom- und Mangansalz-Lösung mit Alkalilauge oder Ammoniak und anschließendes Auswaschen der löslichen Anteile mit Wasser erfolgen. Als Chrom- und Mangansalze kommen insbesondere die Sulfate, Chloride, Acetate und/oder Nitrate der genannten Elemente in Betracht. Die Abscheidung des Chroms und Mangans auf den Katalysatorträger kann auch als Ammonium-Mangan-Chromat oder Ammonium-Alkali-Mangan-Chromat aus einer Lösung von Mangan(II)-Salzen und Ammoniumbichromat mittels Ammoniak und/oder basischer Alkaliverbindungen erfolgen. Besonders gleichmäßige und festhaftende Abscheidungen erhält man, wenn die Basenzugabe langsam und gleichmäßig unter Vermeidung größerer Konzentrationsunterschiede erfolgt. Hierzu kann beispielsweise die Fällung mittels Harnstoff unter hydrolysierenden Bedingungen vorgenommen werden, wodurch die Bedingungen der langsamen Basenzugabe besonders gut gewährleistet sind.

Nach dem Aufbringen der Chrom- und Manganverbindungen und der beschriebenen Ausfällung wird der so beaufschlagte Katalysatorträger frei von löslichen Verbindungen gewaschen, bevor er auf höhere Temperaturen (etwa 200-450°C, bevorzugt 250-350°C) erhitzt wird. Nach dieser Temperung ist der mit Chrom und Mangan beaufschlagte Träger bereit zur Tränkung mit den übrigen genannten Katalysatorbestandteilen.

Die Tränkung des Trägers mit den Edelmetallen bzw. mit Alkalihydroxid und Alkalisulfat (jeweils eines oder mehrere von diesen) erfolgt getrennt. Hierbei kann so vorgegangen werden, daß zunächst die Edelmetalle, beispielsweise in Form wäßriger Lösungen ihrer Chloride, Nitrate, Acetate oder anderer geeigneter Salze auf den Träger aufgetränkt werden, wobeinach einer Trocknung eine weitere Tränkung mit einer Alkalihydroxidlösung und einer Alkalimetallsulfatlösung erfolgt. Bei dieser Behandlung werden die Edelmetalle in Form ihrer Oxide oder Hydroxide ausgefällt. Das Auftränken des oder der Alkalihydroxide und des oder der Alkalimetallsulfate kann getrennt oder gemeinsam erfolgen. Nach einer abschließenden Trocknung steht der erfindungsgemäße Katalysator zur Verfügung. Er wird in bevorzugter Weise im Reaktor vor seinem Einsatz durch Behandlung mit Wasserstoff bei erhöhter Temperatur, wie bei 120-400°C, bevorzugt bei 150-380°C, aktiviert.

Man kann jedoch auch den Träger zunächst mit einer Alkalihydroxidlösung tränken, anschließend trocknen und auf den so vorbehandelten basisch eingestellten Katalysatorträger die genannten Salze der Edelmetalle auftragen, wobei im Moment der Tränkung auch die Ausfällung der Edelmetalle in Form ihrer Oxide oder Hydroxide erfolgt. Bei dieser Variante kann das zusätzliche Auftränken eines oder mehrerer Alkalimetallsulfate gemeinsam mit dem Alkalihydroxid, vor oder nach dem Auftragen des Alkalihydroxids oder als abschließende Tränkung nach dem Auftragen der Edelmetalle erfolgen. Auch hier erfolgt nach jedem Auftränken ein separates Trocknen. Nach der abschließenden Trocknung ist auch nach dieser Variante der Katalysator einsatzbereit und kann in der beschriebenen Weise vorab mit Wasserstoff bei erhöhter Temperatur aktiviert werden.

Anstatt den genannten Träger zur Beaufschlagung mit den genannten Stoffen zu tränken, kann er auch mit geeigneten Lösungen besprüht werden. Die hierzu erforderlichen Arbeitsgeräte und die Einstellung der gewünschten Beaufschlagung durch Wahl der Menge und Konzentration der Lösungen der genannten Elemente sind dem Fachmann im Prinzip bekannt.

Neben wäßrigen Lösungen kommen grundsätzlich auch alkoholische Lösungen oder Lösungen in niederen Carbonsäuren oder niederen Aminen in Frage, sofern die vorgesehenen Salze der Edelmetalle bzw. die basischen Alkalimetallverbindungen darin löslich sind.

Die erfindungsgemäßen Katalysatoren eignen sich hervorragend zur Dehydrierung von gegebenenfalls substituiertem Dicyclohexylamin zu gegebenenfalls substituiertem Diphenylamin, wobei hervorragend hohe Aktivitäten und Selektivitäten beobachtet werden.

Die Erfindung betrifft daher weiterhin ein Verfahren zur Herstellung von Diphenylamin der Formel

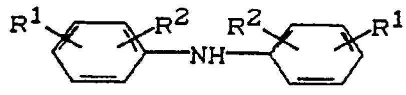

(I),

in der $R^1$ und $R^2$ unabhängig voneinander Wasserstoff, $C_1$-$C_4$-Alkyl oder $C_1$-$C_4$-Alkoxy bedeuten, das dadurch gekennzeichnet ist, daß man Dicyclohexylamine der Formel

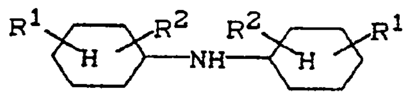

(II),

in der $R^1$ und $R^2$ die obige Bedeutung haben, an einem Rhodium-Katalysator der oben beschriebenen Art bei 250-450°C und 1-20 bar behandelt.

Die Reste $R^1$ und $R^2$ haben unabhängig voneinander die Bedeutung von Wasserstoff, $C_1$-$C_4$-Alkyl oder $C_1$-$C_4$-Alkoxy. Beispiele für die genannten Alkyl- bzw. Alkoxy-Substituenten sind : Methyl, Ethyl, Propyl, Isopropyl, Butyl, Isobutyl, Methoxy, Ethoxy, Propoxy, Isopropoxy, Butoxy oder Isobutoxy. In bevorzugter Weise haben die genannten Substituenten 1-2 C-Atome, besonders bevorzugt handelt es sich um Methyl bzw. Methoxy. In weiterhin bevorzugter Weise hat einer der Substituenten $R^1$ und $R^2$ die Bedeutung Wasserstoff, während der andere Substituent Alkyl bzw. Alkoxy im genannten Umfang bedeutet. In besonders bevorzugter Weise richtet sich das Verfahren auf die Herstellung von nicht-substituiertem Diphenylamin.

Das erfindungsgemäße Verfahren wird bei einer Temperatur von 250-450°C, bevorzugt 300-400°C, und einem Druck von 1-10 bar, bevorzugt 1-6 bar, in der Gasphase durchgeführt. In einer dem Fachmann bekannten Weise werden im allgemeinen niedrigere Temperaturen im genannten Bereich niedrigeren Drücken des ebenfalls offenbarten Bereiches zugeordnet und umgekehrt, so daß das Reaktionsgemisch in der Gasphase bleibt.

Das umzusetzende, gegebenenfalls substituierte Dicyclohexylamin kann selbstverständlich als solches erfindungsgemäß eingesetzt werden. Es ist jedoch von besonderem Vorteil, daß das Dicyclohexylamin auch im Gemisch mit anderen Stoffen eingesetzt werden kann. Solche anderen Stoffe sind beispielsweise zusätzlich vorhandenes, gegebenenfalls substituiertes Cyclohexylamin oder ein Gemisch von zusätzlich vorliegendem, gegebenenfalls substituiertem Cyclohexylamin und entsprechend substituiertem N-Cyclohexylanilin. Des weiteren kann gegebenenfalls substituiertes Anilin vorliegen, welches beispielsweise bei der Herstellung des Dicyclohexylamins nicht vollständig umgesetzt wurde.

Das Dicyclohexylamin oder sein Gemisch mit einem oder mehreren der genannten Stoffe wird vorteilhaft mit Hilfe eines inerten Trägergasstroms an den Rhodium-Katalysator gebracht. Solche inerten Trägergase sind beispielsweise Stickstoff, Wasserstoff, Argon, niedrige Kohlenwasserstoffe, wie Methan oder Ethan und andere oder Gemische aus diesen Trägergasen. In bevorzugter Weise werden Stickstoff oder Wasserstoff oder ein Gemisch von ihnen als inertes Trägergas eingesetzt. Ein restlicher Gehalt von Ammoniak, beispielsweise aus der Herstellungsstufe des Dicyclohexylamins, ist gleichfalls unschädlich für das erfindungsgemäße Verfahren. Das Trägergas wird in einer Menge von 1-100 l/g Ausgangsmaterial, bevorzugt 1-50 l/g Ausgangsmaterial, eingesetzt. Die Katalysatorbelastung wird mit 0,01-1 kg Ausgangs material je Liter Katalysator und Stunde festgesetzt.

Die Unschädlichkeit von Gemischbestandteilen des Dicyclohexylamins in der oben geschilderten Art erlaubt einer vorteilhaften Variante den Einsatz eines Gemisches, welches sich aus der hydrierenden Alkylierung von Anilin mit Wasserstoff ergibt. In besonderer Form handelt es sich hierbei um eine hydrierende Alkylierung von Anilin mit Wasserstoff bei 150-220°C an einem Ruthenium-Katalysator. In besonders bevorzugter Weise handelt es sich dabei um die hydrierende Alkylierung von Anilin an einem Ruthenium und Palladium enthaltenden Katalysator auf einem Träger, bevorzugt einem Träger aus der Gruppe $Al_2O_3$ und Aluminium-Spinell, der die Edelmetalle in einer Gesamtmenge von 0,05-5 Gew.-%, bevorzugt 0,1-4 Gew.-%, besonders bevorzugt 0,1-3 Gew.-%, und in einem Gewichtsverhältnis Ruthenium zu Palladium wie 1 : 9-9 : 1, bevorzugt 2 : 8-8 : 2, besonders bevorzugt 3 : 7-7 : 3, enthält und der weiterhin 0,1-10 Gew.-%, bevorzugt 0,2-5 Gew.-% einer alkalisch reagierenden Alkalimetallverbindung enthält, wobei alle Prozentsätze auf das Gesamtgewicht des Ruthenium enthaltenden Katalysators bezogen sind.

Solche Katalysatoren sind hauptsächlich durch die Kombination von Ruthenium mit Palladium ausgezeichnet und haben gegenüber den nur Ruthenium enthaltenden Katalysatoren eine beträchtlich erhöhte Standzeit, was für ihren Einsatz im technischen Verfahren unerläßlich ist.

Als alkalisch reagierende Alkalimetallverbindungen für diese Ruthenium und andere Platinmetalle enthaltenden Katalysatoren seien beispielsweise genannt : die Oxide, Hydroxide, Alkoholate oder Salze schwacher Säuren von Lithium, Natrium, Kalium, Rubidium oder Caesium, bevorzugt die Hydroxide, Alkoholate und Salze schwacher Säuren von Lithium, Natrium oder Kalium, besonders bevorzugt von Natrium oder Kalium. Schwache Säuren sind beispielsweise Kohlensäure, Essigsäure, Ameisensäure und andere Carbonsäuren, deren Alkalisalze alkalisch reagieren, und sind in jedem Falle solche, die frei sind von Stickstoff, Halogen, Schwefel und anderen als Gifte für Hydrierkatalysatoren geltenden Elementen. Alkoholate sind beispielsweise solche des Methanols, Ethanols, Propanols, Butanols und anderer Alkohole.

$Al_2O_3$ oder Aluminium-Spinelle als bevorzugte Träger für solche Ruthenium-Katalysatoren sind die gleichen wie die oben offenbarten. Die Herstellung dieser Ruthenium-Katalysatoren kann durch getrenntes Auftragen der Edelmetalle und der alkalisch reagierenden Alkalimetallverbindungen in einer ganz ähnlichen Weise vorgenommen werden, wie dies oben für die Rhodium-Katalysatoren geschildert worden ist.

Eine solche hydrierende Alkylierung von Anilin mit Wasserstoff bei 150-220°C an Ruthenium-Katalysatoren wird bei einem Druck von 0,5-10 bar, bevorzugt 0,5-4 bar, besonders bevorzugt 0,7-2 bar, durchgeführt. Die Katalysatorbelastung beträgt hierbei 0,05-2 kg, bevorzugt 0,1-0,5 kg, Anilin pro Liter Katalysator und Stunde. Der Anteil an Dicyclohexylamin wird erhöht, wenn eine niedrigere Temperatur innerhalb des genannten Bereiches eingestellt wird ; diese Erkenntnis hat Bedeutung für den direkten Einsatz eines solchen Alkylierungsgemisches mit hohem Gehalt an Dicyclohexylamin für die Erfindungsgemäße Herstellung von Diphenylamin.

Der Druckbereich für die hydrierende Alkylierung von Anilin mit Wasserstoff an einem Ruthenium-Katalysator überlappt weitgehend mit dem Druckbereich der erfindungsgemäßen Herstellung von Diphenylamin. Dies erlaubt in einer weiterhin vorteilhaften Variante die Durchführung der Stufen der hydrierenden Alkylierung von Anilin und der Dehydrierung des in einem solchen Alkylierungsgemisch vorhandenen Dicyclohexylamins erfindungsgemäß zum Diphenylamin in einem Reaktor (oder zwei unmittelbar hintereinandergeschalteten Reaktoren) in der Weise, daß gegebenenfalls substituiertes Anilin, Wasserstoff und die nachstehend genannten Recyclisierungsstoffe über zwei hintereinanderliegende Katalysatorschichten geleitet werden, deren erste einen Ruthenium-Katalysator enthält und bei einer Temperatur von 150-220°C gehalten wird und deren zweite einen Rhodium-Katalysator enthält und bei 250-450°C gehalten wird.

Bei dieser vorteilhaften Variante bildet sich aus Anilin und Wasserstoff in der ersten Stufe Dicyclohexylamin, Cyclohexylamin und N-Cyclohexyl-anilin neben nicht umgesetztem Anilin und Wasserstoff und weiterhin gebildetem Ammoniak. Dieses Gemisch wird ohne Zwischenisolierung der zweiten Katalysatorschicht zugeführt, wobei Wasserstoff und Ammoniak als Trägergas dienen (gegebenenfalls im Gemisch von für die erste Stufe eingesetztem Stickstoff als dort verwendetem Trägergas). In der zweiten Stufe wird in erfindungsgemäßer Weise das Dicyclohexylamin sowie das N-Cyclohexyl-anilin zu Diphenylamin (bzw. den oben offenbarten substituierten Diphenylaminen) um gesetzt. Alle nicht zum Diphenylamin umgesetzten Komponenten des entstehenden Reaktionsgemisches können der Stufe der hydrierenden Alkylierung von Anilin zugeführt werden. Diese Recyclisierung bedeutet eine beträchtliche Verbesserung der Wirtschaftlichkeit des Gesamtverfahrens. Es kann nützlich sein, einen Teil des im Recyclisierungsgemisch vorhandenen Ammoniaks auszuwaschen oder durch Kompression auszukondensieren. Selbstverständlich ist es möglich, das Recyclisierungsgemisch auch noch in anderer Weise, die dem Fachmann grundsätzlich bekannt ist, aufzutrennen und gezielt Anteile aus dem Recyclisierungsgemisch auszuschleusen.

Die vorteilhafte Kombination der hydrierenden Alkylierung von Anilin und der nachfolgenden erfindungsgemäßen Herstellung von Diphenylamin erlaubt weiterhin, die Reaktionswärme der Stufe der hydrierenden Alkylierung in der zweiten Stufe der erfindungsgemäßen Herstellung von Diphenylamin zu verwerten.

Für die genannte Kombination der beiden Reaktionsstufen ist es weiterhin von besonderem Vorteil, wenn in der ersten Stufe der speziell genannte Ruthenium-Katalysator mit einem Gehalt an Palladium und einem Gehalt an der alkalisch reagierenden Alkalimetallverbindung eingesetzt wird, da dieser sehr viel Dicyclohexylamin, viel N-Cyclohexyl-anilin, aber sehr wenig unerwünschte Nebenprodukte wie Cyclohexan und Benzol ergibt.

Beispiel 1

50 g eines mit Chrom und Mangan gemäß EP-Anmeldung 0208933, Beispiel 1, beaufschlagten $\gamma$-$Al_2O_3$ in Form von Pellets wurden in einem Rundkolben mit einer Lösung von 0,66 g $RhCl_3$ und 0,83 g $H_2PtCl_6$ in 15 ml Wasser gleichmäßig getränkt. Die feuchten Katalysatorpellets wurden bei 120°C im Wasserstrahlvakuum getrocknet und danach mit einer Lösung von 1,46 g NaOH in 15 ml Wasser erneut getränkt und wieder getrocknet. Anschließend wurden die Pellets mit einer Lösung von 1,5 g $K_2SO_4$ in 15 ml Wasser nochmals getränkt und wieder getrocknet.

Ein Reaktionsrohr mit einem Durchmesser von 17 mm und einer Länge von etwa 600 mm, dessen oberer

Teil als Verdampfungszone diente und das im unteren Teil mit 30 ml des angefertigten Katalysators gefüllt war, wurde durch eine elektrische Beheizung auf 380°C gehalten. Bei dieser Temperatur wurde der Katalysator zunächst 16 h im $H_2$-Strom aktiviert. Unter Benutzung einer geeichten Dosiervorrichtung wurden innerhalb von 3 Stunden 29,7 g Dicyclohexylamin und 10 l $H_2$/h in das Reaktionsrohr geleitet. Das Reaktionsprodukt wurde kondensiert und gaschromatographisch analysiert. Es ergab folgende Zusammensetzung :

| | |
|---|---|
| Diphenylamin | 92,4 % |
| N-Cyclohexyl-anilin | 3,1 % |
| Anilin | 3,4 % |
| Benzol | 0,4 % |
| Nebenprodukte | Rest |

Beispiel 2

100 g eines mit Mangan und Chrom gemäß EP-Anmeldung 0208933, Beispiel 1, beaufschlagten $\gamma$-$Al_2O_3$ in Kugelform (2 bis 6 mm) wurden mit einer Lösung getränkt, die aus 0,79 g $RhCl_3$, 2,50 g $PdCl_2$, 0,8 g konz. Salzsäure und 34 ml Wasser hergestellt worden war. Die feuchten Katalysatorpellets wurden bei 120°C im Wasserstrahlvakuum getrocknet.

50,5 g der Katalysatorpellets wurden zunächst mit einer Lösung von 2,75 g KOH in 15 ml Wasser getränkt und nach einer Zwischentrocknung mehrmals mit einer Lösung von 1,5 g $K_2SO_4$ in 15 ml Wasser getränkt und erneut bei 120°C getrocknet.

30 ml (27,2 g) des so hergestellten Katalysators wurden unter Benutzung des in Beispiel 1 beschriebenen Reaktionsrohres im Wasserstoffstrom (10 l/h) auf 400°C erwärmt und 20 Stunden bei dieser Temperatur gehalten. Dann wurde die Ofentemperatur gesenkt und die Dehydrierungsreaktion bei 360 bis 380°C durchgeführt. Es wurden 5,1 g Dicyclohexylamin und 10 l Wasserstoff bzw. 10 l Stickstoff zusammen über den Katalysator geleitet. Das Reaktionsprodukt zeigte in Abhängigkeit von der Einsatzdauer des Katalysators folgende Zusammensetzung :

| Betriebsstunden des Katalysators: | 220 | 316 | 388 | 681 | 1016 h |
|---|---|---|---|---|---|
| Diphenylamin | 94,3 | 96,8 | 94,6 | 95,7 | 94,0 % |
| N-Cyclohexylanilin | 0,4 | 0,7 | 0,4 | 0,7 | 1,4 % |
| Anilin | 4,6 | 2,0 | 4,0 | 2,9 | 3,1 % |
| Nebenprodukte | Rest | Rest | Rest | Rest | Rest |
| Trägergas | $H_2$ | $N_2$ | $H_2$ | $N_2$ | $N_2$ |

Beispiel 3

In diesem Beispiel diente Anilin als Ausgangsverbindung für die Herstellung von Diphenylamin. Benutzt wurden hierbei zwei übereinanderstehende Reaktionsrohre (innerer Durchmesser = 17 mm), die jeweils mit verschiedenen Katalysatoren gefüllt waren und auch auf verschiedenen Temperaturen gehalten wurden. In dem ersten (oberen) Reaktionsrohr befanden sich 30 ml eines Katalysators mit Ru (0,5%) und Pd (0,5%) auf $Al_2O_3$, der mit 4% NaOH versetzt worden war.

Diese Katalysatorschicht wurde auf 180°C gehalten.

Dieser Katalysator war wie folgt hergestellt worden :

500 g eines handelsüblichen $\gamma$-$Al_2O_3$ (Kugeldurchmesser : 2-5 mm) mit einer spez. Oberfläche von 350 $m^2$/g wurden mit einer Lösung von 20 g NaOH in 170 ml Wasser getränkt und anschließend getrocknet. 100 g

des so behandelten $Al_2O_3$ wurden mit einer Lösung von 2,5 g $RuCl_3$ und 0,83 g $PdCl_2$ in 30 ml Wasser getränkt, anschließend bei 120°C getrocknet und danach 2 Stunden bei 250°C im Wasserstoffstrom aktiviert.

Das Reaktionsrohr mit dem in dieser Art hergestellten Katalysator war mit einem zweiten Rohr verbunden, in dem sich 30 ml eines Katalysators wie in Beispiel 1 befanden und das auf einer Temperatur von 380°C gehalten wurde. In die so hintereinandergeschalteten Reaktionsrohre wurden im Verlauf von 21,5 h 90 g Anilin zusammen mit 10 l $H_2$/h geleitet. Das aus dem zweiten Reaktionsrohr ausströmende Reaktionsprodukt wurde kondensiert und analysiert. Die Analyse ergab folgende Zusammensetzung :

| | |
|---|---|
| Diphenylamin | 60,4 % |
| Cyclohexylamin | 0,4 % |
| N-Cyclohexyl-anilin | 6,3 % |
| Anilin | 32,3 % |
| Nebenprodukte | Rest. |

Das nach Abtrennung des Diphenylamins verbleibende Gemisch wurde recyclisiert.

Das nicht kondensierte Abgas wurde nach Entfernung eines Teils des $NH_3$ ebenfalls recyclisiert.

## Patentansprüche

1. Rhodium und mindestens ein anderes Platinmetall aus der Gruppe Palladium, Platin und Iridium enthaltende Katalysatoren auf Trägern, die die Edelmetalle in einer Gesamtmenge von 0,05-5 Gew.-%, bevorzugt 0,05-3 Gew.-%, besonders bevorzugt 0,1-2 Gew.-%, und einem Gewichtsanteil des Rhodiums an allen Edelmetallen von 10-90%, bevorzugt 15-80%, besonders bevorzugt 20-70%, enthalten und die weiterhin Zusätze von 1-6 Gew.-% eines Alkalihydroxids und 1-6 Gew.-% eines Alkalimetallsulfats enthalten, wobei alle Prozentsätze auf das Gesamtgewicht des Katalysators bezogen sind.

2. Katalysatoren nach Anspruch 1, dadurch gekennzeichnet, daß der Träger ein $Al_2O_3$ oder ein Aluminiumspinell ist.

3. Katalystoren nach Anspruch 2, dadurch gekennzeichnet, daß der Träger ein mit Chrom und Mangan behandeltes $Al_2O_3$ oder ein so behandelter Aluminiumspinell ist.

4. Verfahren zur Herstellung der Katalysatoren nach Anspruch 1, dadurch gekennzeichnet, daß man einen Träger in getrennten Vorgängen mit einer wäßrigen Lösung der Edelmetalle, die ausreicht, um die genannte Menge an Edelmetall auf den Träger zu bringen, und wäßrigen Lösungen von Alkalihydroxid und Alkalimetallsulfat tränkt und nach jedem Tränkungsvorgang die Katalysatoren trocknet.

5. Verfahren zur Herstellung von Diphenylamin der Formel

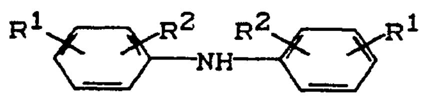

in der $R^1$ und $R^2$ unabhängig voneinander Wasserstoff, $C_1$-$C_4$-Alkyl oder $C_1$-$C_4$-Alkoxy bedeuten, dadurch gekennzeichnet, daß man ein Dicyclohexylamin der Formel

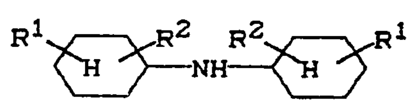

in der $R^1$ und $R^2$ die obige Bedeutung haben, an einem Rhodium-Katalysator nach Anspruch 1 bei 250-450°C, bevorzugt 300-400°C, und 1-20 bar, bevorzugt 1-6 bar, behandelt.

6. Verfahren nach Anspruch 5, dadurch gekennzeichnet, daß man das Dicyclohexylamin im Gemisch mit Cyclohexylamin oder im Gemisch mit Cyclohexylamin und N-Cyclohexyl-anilin einsetzt.

7. Verfahren nach Ansprüchen 5 und 6, dadurch gekennzeichnet, daß man das Dicyclohexylamin oder sein Gemisch mit Cyclohexylamin oder sein Gemisch mit Cyclohexylamin und N-Cyclohexyl-anilin mit Hilfe eines inerten Trägergasstroms an den Katalysator bringt.

8. Verfahren nach Anspruch 6, dadurch gekennzeichnet, daß man ein Gemisch einsetzt, wie es aus der hydrierenden Alkylierung von Anilin mit Wasserstoff bei 150-220°C an einem Ruthenium-Katalysator erhalten wird.

9. Verfahren nach Anspruch 8, dadurch gekennzeichnet, daß man die Stufen der hydrierenden Alkylierung und der Dehydrierung zum Diphenylamin in einem Reaktor mit zwei hintereinanderliegenden Katalysatorschichten durchführt, wobei die erste Katalysatorschicht ein Ruthenium-Katalysator ist und bei 150-220°C gehalten wird und die zweite ein Rhodium-Katalysator ist und bei 250-450°C gehalten wird.

## Claims

1. Catalysts containing rhodium and at least one other platinum metal from the group consisting of palladium, platinum and iridium on supports containing the noble metals in a total amount of 0.05-5% by weight, preferably 0.05-3% by weight, particularly preferably 0.1-2% by weight, the percentage by weight of rhodium with respect to all the noble metals being 10-90%, preferably 15-80%, particularly preferably 20-70%, and furthermore containing additives of 1-6% by weight of an alkali metal hydroxide and 1-6% by weight of an alkali metal sulphate, all percentages being based on the total weight of the catalyst.

2. Catalysts according to Claim 1, characterised in that the support is $Al_2O_3$ or an aluminium spinel.

3. Catalysts according to Claim 2, characterised in that the support is $Al_2O_3$ treated with chromium and manganese or an aluminium spinel thus treated.

4. Process for the preparation of catalysts according to Claim 1, characterised in that a support is impregnated in separate processes with an aqueous solution of the noble metals sufficient to apply the amount of noble metal mentioned to the support, and with aqueous solutions of alkali metal hydroxide and alkali metal sulphate and the catalysts are dried after each impregnation process.

5. Process for the preparation of diphenylamine of the formula

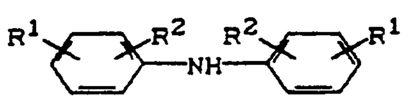

in which $R^1$ and $R^2$ independently of one another denote hydrogen, $C_1$-$C_4$-alkyl or $C_1$-$C_4$-alkoxy, characterised in that a dicyclohexylamine of the formula

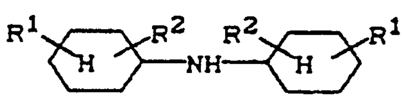

in which $R^1$ and $R^2$ have the above meaning, is treated by passing it over a rhodium catalyst according to Claim 1 at 250-450°C, preferably 300-400°C, and 120 bar, preferably 1-6 bar.

6. Process according to Claim 5, characterised in that dicyclohexylamine is used in a mixture with cyclohexylamine or in a mixture with cyclohexylamine and N-cyclohexyl-aniline.

7. Process according to Claims 5 and 6, characterised in that dicyclohexylamine or its mixture with cyclohexylamine or its mixture with cyclohexylamine and N-cyclohexyl-aniline is passed over the catalyst by means of an inert carrier gas stream.

8. Process according to Claim 6, characterised in that the mixture used is one which has been obtained

from the reductive alkylation of aniline with hydrogen at 150-220°C by passing it over a ruthenium catalyst.

9. Process according to Claim 8, characterised in that the stages of the reductive alkylation and the dehydrogenation to give diphenylamine is carried out in a reactor having two consecutive catalyst beds, the first catalyst bed being a ruthenium catalyst and being maintained at 150-220°C and the second being a rhodium catalyst and being maintained at 250-450°C.

**Revendications**

1. Catalyseurs contenant du rhodium et au moins un autre métal du groupe du platine appartenant a la série du palladium, du platine et de l'iridium sur des supports qui contiennent les métaux précieux dans une quantité totale de 0,05% à 5% en poids, en particulier dans la gamme de 0,05 à 3% en poids et de préférence dans la gamme de 0,1 à 2% en poids et dans une part pondérale du rhodium par rapport à tous les métaux précieux de 10 à 90%, en particulier dans la gamme de 15 à 80%, de préférence dans la gamme de 20 à 70%, et les autres ajouts dans une quantité de 1 à 6% en poids d'un hydroxyde alcalin et 1 à 6% en poids d'un sulfate de métal alcalin, tous les pourcentages se rapportant au poids total du catalyseur.

2. Catalyseurs selon la revendication 1, caractérisés en ce que le support est un $Al_2O_3$ ou un spinelle d'aluminium.

3. Catalyseurs selon la revendication 2, caractérisé en ce que le support est un $Al_2O_3$ traité avec du chrome et du manganèse ou un spinelle d'aluminium ayant subi le même traitement.

4. Procédé de fabrication de catalyseurs selon la revendication 1, caractérisé en ce qu'on imbibe un support dans des procédures séparées avec une solution aqueuse de métaux précieux qui suffit pour amener la quantité donnée de métal précieux sur le support et avec des solutions aqueuses d'hydroxyde alcalin et de sulfate de métal alcalin, et qu'on sèche les catalyseurs après chaque procédure d'imbibition.

5. Procédé de fabrication de diphénylamine de formule

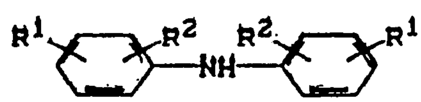

dans laquelle $R^1$ et $R^2$ désignent indépendamment l'un de l'autre de l'hydrogène, un radical alkyle $C_1$-$C_4$ ou alkoxy $C_1$-$C_4$, caractérisé en ce qu'on traite une dicyclohexylamine de formule

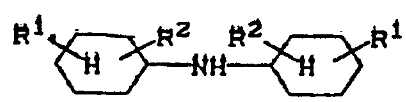

dans laquelle $R^1$ et $R^2$ ont la signification susmentionnée, sur un catalyseur au rhodium selon la revendication 1 à une température de 250 à 450°C, de préférence de 300 à 400°C, et à une pression de 1 à 20 bar, de préférence 1 à 6 bar.

6. Procédé selon la revendication 5, caractérisé en ce qu'on utilise la dicyclohexylamine en mélange avec de la cyclohexylamine ou en mélange avec de la cyclohexylamine et de la N-cyclohexyl-aniline.

7. Procédé selon les revendications 5 et 6, caractérisé en ce qu'on amène sur le catalyseur la dicyclohexylamine ou son mélange avec la cyclohexylamine ou son mélange avec la cyclohexylamine et la N-cyclohexylaniline à l'aide d'un flux de gaz porteur inerte.

8. Procédé selon la revendication 6, caractérisé en ce qu'on utilise un mélange tel qu'il est obtenu à partir de l'alkylation hydrogénante de l'aniline avec de l'hydrogène à une température de 150 à 220 °C sur un catalyseur au ruthénium.

9. Procédé selon la revendication 8, caractérisé en ce qu'on effectue les phases d'alkylation hydrogénante et de déshydrogénation en diphénylamine dans un réacteur avec deux couches successives de catalyseurs, la première couche de catalyseur étant un catalyseur au ruthénium maintenu à une température de 150 à 220°C et la deuxième un catalyseur au rhodium maintenu à une température de 250 à 450°C.